(19) Europäisches Patentamt
European Patent Office
Office européen des brevets

(11) **EP 4 295 369 B1**

(12) **EUROPEAN PATENT SPECIFICATION**

(45) Date of publication and mention
of the grant of the patent:
**25.12.2024   Bulletin 2024/52**

(51) International Patent Classification (IPC):
**G16H 10/20** (2018.01)   **G16H 70/40** (2018.01)
**G16H 50/70** (2018.01)   **G16H 50/50** (2018.01)

(21) Application number: **22705383.2**

(22) Date of filing: **08.02.2022**

(52) Cooperative Patent Classification (CPC):
**G16H 10/20; G16H 50/50; G16H 50/70;
G16H 70/40**

(86) International application number:
**PCT/EP2022/052966**

(87) International publication number:
**WO 2022/175131 (25.08.2022 Gazette 2022/34)**

(54) **SYSTEM AND METHOD FOR MEASURING THE TREATMENT EFFECT OF A DRUG**

SYSTEM UND VERFAHREN ZUR MESSUNG DER BEHANDLUNGSWIRKUNG EINES
ARZNEIMITTELS

SYSTÈME ET PROCÉDÉ POUR MESURER L'EFFET DE TRAITEMENT D'UN MÉDICAMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.02.2021   EP 21158462
10.03.2021   EP 21161795**

(43) Date of publication of application:
**27.12.2023   Bulletin 2023/52**

(73) Proprietors:
• **Boehringer Ingelheim International GmbH
55216 Ingelheim am Rhein (DE)**
• **BI X GmbH
55216 Ingelheim am Rhein (DE)**

(72) Inventors:
• **BROCKSTEDT, Ekkehard
55216 Ingelheim am Rhein (DE)**
• **ABUSNINA, Ali
55216 Ingelheim am Rhein (DE)**
• **LINK, Jasmin
55216 Ingelheim am Rhein (DE)**
• **PEREZ-PITARCH, Alejandro
55216 Ingelheim am Rhein (DE)**

• **SCHWARZ, Philipp
55216 Ingelheim am Rhein (DE)**
• **STOCK, Christian
55216 Ingelheim am Rhein (DE)**

(74) Representative: **Bittner, Peter et al
Peter Bittner und Partner
Herrenwiesenweg 2
69207 Sandhausen (DE)**

(56) References cited:
**WO-A1-2020/072548      US-A1- 2015 315 651
US-A1- 2019 220 733**

• **YU ROSIE Z ET AL: "Prediction of clinical
responses in a simulated phase III trial of Crohn's
patients administered the antisense
phosphorothioate oligonucleotide ISIS 2302:
comparison of proposed dosing regimens",
ANTISENSE & NUCLEIC ACID DRUG
DEVELOPMENT, MARY ANN LIEBERT, INC., NEW
YORK, US, vol. 13, no. 1, 1 February 2003
(2003-02-01), pages 57 - 66, XP002552080, ISSN:
1087-2906, DOI: 10.1089/108729003764097340**

## Description

The present invention generally relates to electronic data processing, and more particularly, relates to computer-implemented methods, computer program products and systems for measuring the treatment effect of a drug with improved reliability.

## Background

When a drug (medicament) is developed for medical treatment of human patients having a particular disease, it is important to measure the treatment effect of the drug before applying it at a large scale to patients to ensure that effective treatment will be applied to said patients so that the disease can be cured or at least the progression of the disease can be positively influenced. To measure the treatment effect of the drug, clinical studies (clinical trials) are conducted in multiple stages with the last stage typically involving a large number of participants in the respective clinical trial.

A clinical target trial for measuring the treatment effect of the drug always involves at least one so-called treatment arm with human patients receiving experimental treatment with said drug, and a control arm with patients not receiving experimental treatment but instead are treated with standard-of-care (SOC) while using placebos. It may occur that the target trial is unbalanced with regard to the participants in that not all relevant characteristics of patients are equally represented in the target trial. There may be an imbalance in age, sex, in the number of participants with particular known previous illnesses, etc.

Such imbalances can lead to lower reliability of the measurement of the treatment effect based on the results obtained from the target trial.

Further, it is to be noted that the participation in a target trial typically involves a burden for the participants which goes beyond SOC. Typically, patients in such a trial have to undergo certain tests, receive injections or the like, etc. In particular, patients which belong to the control arm of the target trial do not even receive any experimental treatment but only receive placebos instead of the drug. In other words, patients in the control arm may experience an additional psychological as well as physical burden although their health state is not positively affected by their trial participation.

US application 2019/220733 A1 discloses simulator engines to simulate patient populations, disease progressions, and/or predicted responses to various treatments using a sampling engine for drawing samples from generative models that simulate the probability distribution of the data. A Boltzmann machine is trained to simulate patients with a disease. This model can then be used to create a cohort of simulated patients that match the inclusion criteria of new trial. Data from simulated patients can be used to supplement data from a concurrent placebo arm using standard statistical methods.

In "Prediction of clinical responses in a simulated phase III trial of Crohn's patients administered the antisense phosphorothioate oligonucleotide ISIS 2302: comparison of proposed dosing regimens", Antisense Nucleic Acid Drug Dev. 2003 Feb;13(1):57-66, Rosie Yu et al. disclose a method for simulated patient population where the patient population for the simulated active treatment arm was selected 384 Crohn's patients enrolled in earlier phase II trials. To provide a placebo control arm with realistic clinical responses, a bootstrap approach is used.

PCT application WO 2020/072548 A1 discloses a method for enrolling patient candidates in a clinical trial for a treatment where the clinical trial for a treatment where the clinical trial data includes measures of patient condition associated with the conduct of the clinical trial and addresses the problem of extraordinary challenges for drug discovery and drug development companies trying to conduct human clinical tries due to the disease progression heterogeneity.

US application 2015/315651 A1 discloses a method of conducting a randomized clinical trial by detecting a genotype of a placebo-associated polymorphism in a subpopulation of human subjects, wherein the human subjects are candidates for a clinical trial, thereby providing a first sub-group of subjects comprising a first genotype of the polymorphism and a second sub-group of subjects comprising a second genotype of the polymorphism, wherein the first and second genotypes are not the same, and distributing the first sub-group evenly, unevenly and/or randomly into at least a first study group and a second study group, wherein at least the first study group is administered a first treatment and the second study group is administered a placebo treatment.

## Summary

There is therefore a need to provide system and method for improving the measurement of the treatment effect of a medicament (medical drug) while at the same time providing some relief in particular with regard to the suffering of patients in the control arm of the respective target trial. It is thereby advantageous to achieve such an improvement in particular in situations where the number of participants in a target trial is lower than desired.

This technical problem is solved by the embodiments - a computer-implemented method, a computer program product, and a computer system - for measuring the treatment effect of a drug as claimed in the independent claims.

In one embodiment, a computer system is provided for measuring the treatment effect of a medical drug for a disease. A medical drug, as used herein, is a substance used in the diagnosis, treatment, or prevention of a disease or as a component of a medication. In general, the treatment effect of a drug is measured as the difference in change from baseline of a measured clinical outcome between the respective treatment arm and control arm. The system has one or more interfaces which are configured to establish a communication with other computer systems, such as for example, remote data storage systems, remote data analytics systems, or the like. Via a respective interface, the system obtains a disease progression model of the disease which is targeted by the experimental drug.

The disease progression model is based on historical covariates and clinical outcome data reflecting the progression of the disease for a plurality of patients affected by said disease, and quantitively describes the time course of disease progression by one or more corresponding covariates. A clinical outcome is a measure referring to the occurrence of a disease, symptom, sign or laboratory abnormality, which constitutes the target outcome of clinical trials. That is, the term "clinical outcome" refers to the measured variable (e.g., peak volume of oxygen, PROMIS Fatigue score, etc.) or an event such as death, hospitalization, disease progression, etc.

For instance, the clinical outcome in oncology trials may be progression free survival, defined as the time since the treatment started until the disease progressed or the patient died, or it may be overall survival, defined as the time since the treatment started until the patient died. Another example may be in a disease affecting the lungs, the clinical outcome may be the loss of functional capacity of the lungs measured as the difference in volume that a patient can exhale at the beginning of the trial and at the end of the trial. Examples of further clinical outcomes currently accepted by the EMA (cf. European Medicines Agency. Clinical efficacy and safety guidelines. Available from: https://www.ema.europa.eu/en/human-regulatory/research-development/scientific-guidelines/clinical-efficacy-safety-guidelines) or FDA (cf. U.S. Food & Drug Administration. Clinical Outcome Assessment Compendium. Available from https://www.fda.gov/drugs/development-resources/clinical-outcome-assessment-compendium) are given in the following Table 1:

Table 1: Examples of clinical outcomes accepted by EMA/FDA guidelines

| Indication | Clinical Outcome |
| --- | --- |
| Inflammatory bowel disease (IBD) such as Ulcerative Colitis (UC) | Clinical Remission assessed by Mayo component sub-scores at week X (Time Frame: X weeks; 12 weeks for induction / 52 weeks for remission) |
| Interstitial lung disease (ILD) such as Systemic sclerosis (SSc) | annual rate of decline in FVC over X weeks in patients with SSc-ILD (Time Frame: up to week (wk) 52 after the start of administration) |
| Nonalcoholic Steatohepatitis (NASH) with compensated Cirrhosis | Improvement in fibrosis by at least 1 stage with no worsening of NASH, using NASH Clinical Research Network (CRN) scoring system (Time Frame: 18 months) |
| Wet-age-related macular degeneration (wAMD) | Change from Baseline in Best-Corrected Visual Acuity (BCVA) Score at the Average of Week 36 and Week 40, as Assessed Using the Early Treatment Diabetic Retinopathy Study (ETDRS) Visual Acuity Chart at a Starting Distance of 4 Meters (Time Frame: Baseline to Week 40) |
| Colorectal cancer (CRC) | Progression-free survival (PFS) [Time Frame: X (e.g. 2) years] |

Covariates may include one or more of the following: one or more trends of the development of disease symptoms over time, the variability in disease progression between patients, and a quantitative description of the relationship between patient characteristics, disease characteristics, treatment characteristics and the disease progression dynamics.

In general, a disease progression model is developed using available historical data. Disease progression models are mathematical functions used to quantitatively describe the time course of disease progression (e.g., size of tumor over time, progression of disability scores over time, functional markers evolution over time). In the publication Cook, S.F., Bies, R.R. Disease Progression Modeling: Key Concepts and Recent Developments. Curr Pharmacol Rep 2, 221-230 (2016), disease modeling techniques are disclosed involving the use of mathematical functions to describe quantitatively the time course of disease progression, Disease progression models can be more or less comprehensive, and can describe aspects such as:

- the disease progression trends observed over time (e.g. linear, exponential),
- the variability in disease progression between patients,
- a quantitative description of relationship between patient characteristics (e.g. sex, age, weight, comorbidities, smoking status, ethnic group),

- disease characteristics (e.g. severity of the disease, time since diagnosis),
- treatment characteristics (e.g. drug, dose, schedule, concomitant medications), and
- the disease progression dynamics.

The aspects included in the disease progression model are referred to as the relevant covariates herein.

The historical data used to develop such a model may be data coming from a previously conducted clinical trial, multiple clinical trials, electronic health records, data registries, or other sources.

Disease progression models are available for a plurality of diseases. For example, "Wu K, Gamazon ER, Im HK, Geeleher P, White SR, Solway J, et al. Genome-wide interrogation of longitudinal FEV1 in children with asthma. Am J Respir Crit Care Med. 2014;190(6):619-27" describe a disease progression model for asthma with the clinical outcome "forced expiratory volume in 1 s" (FEV1) and the relevant covariates "age" and "height" of the patients. Another example is a disease progression model for melanoma described by M S Chatterjee et al. in "Population Pharmacokinetic/Pharmacodynamic Modeling of Tumor Size Dynamics in Pembrolizumab-Treated Advanced Melanoma. CPT Pharmacometrics Syst. Pharmacol. (2017) 6, 29-39" with the clinical outcome "tumor size" and the relevant covariates "number of target lesions" and "ECOG status" of the patients. Yet another example for a Schizophrenia disease progression model is given by Venkatesh Pilla Reddy et al. in "Pharmacokinetic-pharmacodynamic modeling of antipsychotic drugs in patients with schizophrenia Part I: The use of PANSS total score and clinical utility. Schizophrenia Research, Volume 146, Issues 1-3, 2013, Pages 144-152". The clinical outcome in this example is "PANSS total score" and the relevant covariates are "treatment" and "placebo effect". Yet another example for a disease progression model to predict tumor size over time (clinical outcome) depending on the treatment (covariate) taken by the patients is disclosed by Mario Nagase et al. in "Modeling Tumor Growth and Treatment Resistance Dynamics Characterizes Different Response to Gefitinib or Chemotherapy in Non-Small Cell Lung Cancer; CPT Pharmacometrics Syst. Pharmacol. (2020) 9, 143-152."

Further examples of disease progression models are described in the following papers. Lu et al. describe in" Population Analysis of wet-AMD Disease Progression and The Therapeutic Effect of Ranibizumab" (Poster presented at the Population Approach Group in Europe (PAGE) 21st Annual Meeting, June 5-8, 2012, Venice, Italy, available at https://www.page-meeting.org/pdf_assets/8526-2012%20PAGE%20poster version_FINAL.PDF) a disease progression model for wAMD, where the clinical outcome is visual acuity and the covariate is exposure to drug.

A disease progression model for CRC with the clinical outcome being tumor size and survival and the covariate being exposure to drug is described by Claret et al. in "Model-based prediction of phase III overall survival in colorectal cancer on the basis of phase II tumor dynamics. J Clin Oncol. 2009 Sep 1;27(25):4103-4108. doi: 10.1200/JCO.2008.21.0807. Epub 2009 Jul 27. PMID: 19636014."

A disease progression model for AMD or DME with visual acuity as clinical outcome and exposure to drug as the predictive covariate is described by Basile et al. in "Integrating disease progression models, non-clinical pharmacokinetic data and treatment response endpoints to optimize intravitreal dosing regimens. Int J Clin Pharmacol Ther. 2014 Jul;52(7):574-586. doi: 10.5414/CP201998. PMID: 24755127".

A person skilled in the art is able to identify or develop further disease progression models which are suitable in the context of the herein disclosed approach in the literature.

Further, the system has an interface to receive a target trial dataset (also referred to as the trial data set herein) with trial covariates and clinical outcome data obtained from a plurality of human patients participating in a target trial. The target trial includes one or more treatment arms with human patients receiving experimental treatment, and a control arm with patients not receiving experimental treatment.

Further, the system has an artificial patient generator module to create an artificial patient dataset. There are two alternative ways how the artificial patient generator can create artificial patient data records.

In one implementation, it uses distributions of one or more corresponding covariates. Relevant covariates are identified from the disease progression model that was developed based on historical data (e.g., the result from earlier trial). Then, a mathematical description of the distribution of relevant covariates, and the correlation between them is built based on the target trial. For instance, if the disease progression model considers height and weight to be the covariates relevant to how the disease will progress over time in a particular population, a mathematical description of the distribution of these two covariates (e.g., mean, median, standard deviation) and their correlation (for the covariates age and height most likely a positive correlation is found, meaning the taller a patient is the heavier he/she will be) can be used to generate a dataset describing these covariates. This mathematical description is then used to sample a large artificial patient dataset (e.g., 10000 patients), with artificial patients which are virtually impossible to distinguish from the real patients. Each data record in the generated artificial patient dataset corresponds to a simulated patient. Then, the artificial patient dataset is filtered in accordance with inclusion-exclusion criteria of the target trial so that data records associated with artificial patients which do not meet the inclusion-exclusion criteria of the target trial are removed from the artificial patient dataset. That is, simulated patients which do not meet the inclusion criteria, or which meet the exclusion criteria of the target trial, are then removed from the artificial patient dataset, trying to replicate a screening process (e.g., the target trial does not recruit patients with weights above 130 kg and, therefore, such artificial patient data records are removed from the artificial patient dataset.) Finally, this process results in a dataset where each data record represents an artificial patient with the characteristics of the patient, the characteristics of the patient's disease and the treatments the patient is receiving - to the extent they are considered relevant in the used disease progression model. It is to be noted that a person skilled in the art will understand the term inclusion-exclusion criteria of a target trial as comprising criteria which qualify a patient to participate in the target trial (inclusion criteria) as well as comprising criteria which disqualify a patient from participating in the target trial (exclusion criteria).

In an alternative implementation, the artificial patient dataset is generated by using a bootstrapping approach which includes random sampling with replacement. If the target trial dataset is large enough, this sampling with replacement method may be used. Inherent to this method is that only artificial patients meeting the inclusion/exclusion criteria of the target trial are generated because only such patients are included in the target trial data. Therefore, a filtering step as in the first implementation is not necessary here (although it would not harm because no data would be filtered from the resampled dataset). For example, patients may be randomly sampled from the trial dataset, obtaining paired values of covariates (e.g., age and height), to generate a dataset of covariates for the artificial patients. A large dataset may be generated (e.g., 10000 patients). In order to obtain such a big dataset out of a smaller set of patients sampling with replacement is performed (patients can be sampled more than once).

The artificial patient dataset is then provided as an input to the disease progression model to generate a simulated dataset with simulated covariates and simulated clinical outcome data for simulated artificial patients not receiving experimental treatment. In other words, the simulated artificial patients reflect patients which only receive SOC in the control arm of a target trial. That is, such a simulated patient can play the role of a patient in the control arm of the target trial to relieve a real human patient from the above-mentioned burden of trial participation while not receiving experimental treatment.

At this point the system has: 1) real data for the treatment and control arms of the target trial, and 2) simulated data for artificial patients in the control arm with matching characteristics to those recruited in the target trial obtained by using the disease progression model created based on historical data. These two sources are now brought together into a single analysis. This is performed by a trial analyzer module of the system which determines the treatment effect of the drug by analyzing the trial dataset and the simulated dataset together. To do this, the trial analyzer uses a power prior module to incorporate the simulated dataset as prior information. For example, the power prior approach as described by Ibrahim JG, Chen MH, Gwon Y, Chen F. The power prior: theory and applications. Stat Med. 2015 Dec 10; 34(28):3724-49 may be used to incorporate the simulated patient data as prior information. Thereby, the simulated dataset is given a weight in comparison to the control arm of the trial dataset to avoid overweighting the influence of the artificial patients on the overall result of the analysis. In other words, choosing an appropriate weight to the simulated data is important given that the number of simulated patients can be disproportionally large (e.g., 10000 patients) as compared to the actual number of patients in the target trial (e.g., 300 in treatment arm and 300 in control arm. This weight is based on at least a maximum weight value received by the system from a user. That is, a user of the system provides a limitation for the impact of the artificial patient data on the computation of the treatment effect of the drug. The weight can be interpreted as the number of real patients that would have to be added to the trial to provide the same amount of information as the virtual (artificial) patients will do. For example, if 10000 simulated patients were created but only 100 real patients would have to be added to the control arm, then each simulated patient would only be taken into account with a 1% weight so that the entire weight of the 10000 simulated patients corresponds to a total number of 100 patients.

The maximum weight value received from the user may be based on any of the following:

- Number of patients used to develop the disease progression model. This number of (real) patients in the respective historic trial data corresponds to an upper limit for a meaningful maximum weight value provided by the user. A higher weight value would allow an impact on the power prior analysis which cannot be justified because it would pretend a higher reliability of the disease progression model than the actual reliability achieved based on the historical data.

- Similarity of patients used to develop the disease progression model and patients expected to be enrolled in the target trial, and assumptions made to extrapolate from one to another population. For example, disease progression model may have been developed using historic data from adult patients only. For example, adult patients are not as valuable to inform a pediatric trial as pediatric patients would be. A correction factor can be applied to account for an assumption made that the relation between certain combinations of covariates and a respective clinical outcome holds also for pediatric patients. For example, the maximum number of patients may be reduced to an appropriate percentage of the patients used to develop the disease progression model (e.g., only 50% of these patients).

- Sensitivity analysis: a false positive results probability (probability of concluding that the drugs works when it does not) is considered as the worst-case scenario. For example, simulated patients could progress faster than the real patients recruited in the trial. As a consequence, when pooling together the artificial and the real patient data, the progression rate of the disease in control patients could be estimated to be faster than the true progression rate. When comparing control and treatment arms, this would result in an increased probability of concluding the drug slows down the disease progression when actually it does not. Under this worst-case scenario, a range of weights is evaluated through clinical trial simulation and analysis using the power prior, and the maximum weight. The goal is to keep the probability of a false positive outcome below a predefined threshold. Typically, a 10% probability is accepted by regulators in the pharma sector. As a result of the sensitivity analysis, the maximum weight for the artificial patients may be further reduced.

A person skilled in the art will acknowledge that the maximum weight value received from the user may be further based the following criteria:

- the precision of the estimates of the disease progression model;
- the results of model validation; and
- the size of target trial.

The clinical outcomes for the control arm in the target trial are then updated using the power prior by taking into account the maximum weight value for the artificial patient data. By including the simulated patient data, the distribution of the control arm becomes narrower and higher than the original distribution of the control arm of the target trial. That is, the estimated measure of the treatment effect (i.e., the difference in the clinical outcome progression rate between treatment and control arm) based on the herein disclosed method is of higher reliability than an estimate which is merely based on the target trial data alone. It is to be noted that this improvement is achieved without involving additional real patients in the control arm which would lead to the above-mentioned disadvantages for humans participating in the control arm.

In one embodiment, the weight is further based on the similarity between the clinical outcome data for artificial patients not receiving experimental treatment and the clinical outcome data obtained from the control arm of the target trial. This similarity may be obtained by using a dynamic borrowing method with a higher similarity leading to a higher dynamic borrowing weight value. The weight used for the analysis is then the lower of the received maximum weight value and the obtained dynamic borrowing weight value. In other words, in this embodiment an automatic quantitative algorithm is used together with the maximum weight value to select the weight finally used for measuring the treatment effect. An example of such an algorithm is the dynamic borrowing for power prior analysis described by Ibrahim JG, Chen MH, Gwon Y, Chen F. The power prior: theory and applications. Stat Med. 2015 Dec 10; 34(28):3724-49. This dynamic borrowing algorithm is adapted to the treatment effect analysis by implementing a cost function considering the difference between the observed outcomes in the patients enrolled in the trial and the simulated outcomes of the patients in the simulated patient dataset, and the amount of information borrowed from the simulated dataset. That is, the method uses the already available real patient data and borrows additional information from the simulated patient data. In doing this, a balance is reached between the risk of introducing bias into the analysis and the improvement of the estimate precision.

Further aspects of the invention will be realized and attained by means of the elements and combinations particularly depicted in the appended claims. It is to be understood that both, the foregoing general description and the following detailed description are exemplary and explanatory only and are not restrictive of the invention as described.

**Short description of the figures**

FIG. 1 illustrates a block diagram of a computer system for measuring the treatment effect of a medical drug for a disease according to an embodiment;

FIG. 2 is a simplified flow chart of a computer-implemented method for measuring the treatment effect of a medical drug for a disease according to an embodiment;

FIG. 3 illustrates observed data from a clinical trial used to build a variance covariance matrix, and simulated data created by using the variance covariance matrix;

FIG. 4 illustrates the probability of false positive outcomes with regard to the treatment effect of the drug for different weights given to artificial patients;

FIG. 5 illustrates an example behavior of a cost-function implemented by a dynamic borrowing for power prior algorithm over a range of weights according to an embodiment;

FIG. 6A illustrates an example of a posterior distribution of FEV1 change from a baseline estimate when using artificial patients, and the distribution when not using the artificial patients;

FIG. 6B shows an example distribution of a treatment effect measure with artificial patients in comparison the measure without artificial patients;

FIG. 7 is a table reflecting properties of an example of a particular disease progression model; and

FIG. 8 is a diagram that shows an example of a generic computer device and a generic mobile computer device, which may be used with the techniques described herein.

**Detailed description**

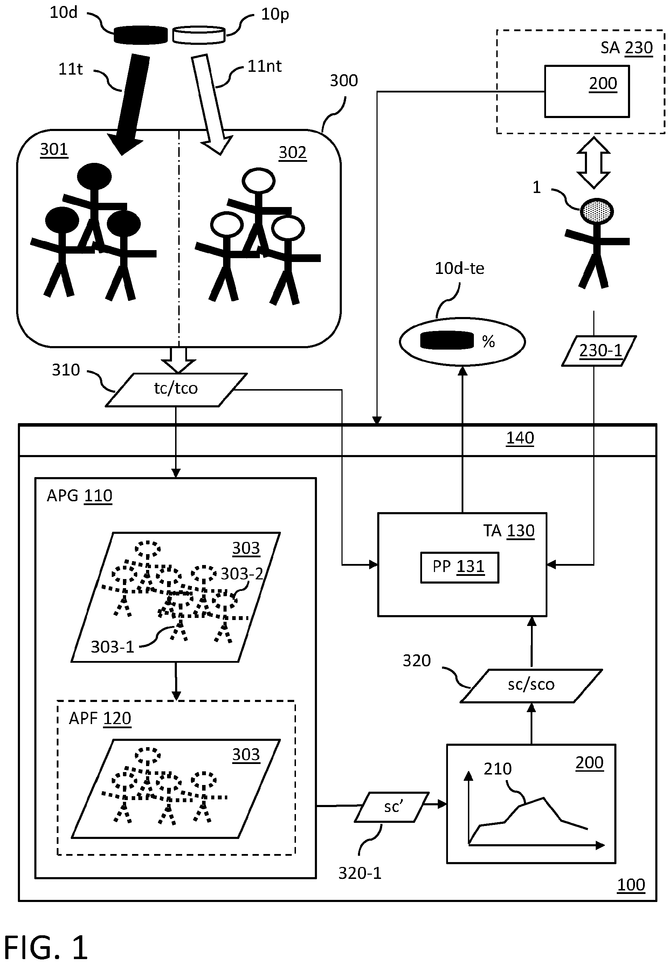

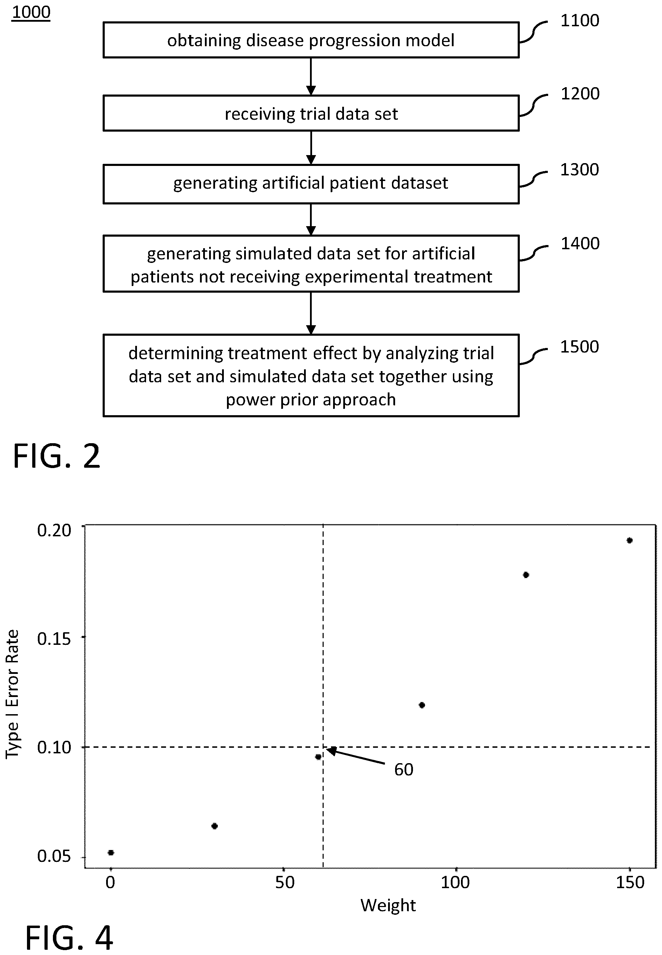

FIG. 1 includes a block diagram of a computer system 100 for measuring the treatment effect of a medical drug 10d for a disease. FIG. 2 is a simplified flow chart of a computer-implemented method 1000 for measuring the treatment effect of the drug 10d for said disease. The method 1000 can be executed by the system 100. For this reason, FIG. 1 is described in view of FIG. 2 and the following description of FIG. 1 also refers to reference numbers used in FIG. 2.

The system 100 has one or more interfaces 140 to communicate with other computer systems (e.g., simulation systems, data storage systems, etc.) for data retrieval, and further to communicate with a human user 1 of the system. Via the interface 140, the system 100 obtains 1100 a disease progression model 200 of the disease. The obtained disease progression model 200 is based on historical covariates and clinical outcome data reflecting the progression of the disease for a plurality of patients affected by said disease. The disease progression model quantitively describes the time course 210 of disease progression by one or more corresponding covariates. In the herein described example, a disease progression model is used which characterizes lung function change over time in patients with asthma. In this example, the progression of the disease is monitored through "Forced Expiratory Volume in 1 second" (FEV1), which increases with age and is decreased in asthmatic patients. Thereby, the loss of functional capacity of the lungs is measured as the difference in volume that the human patient can exhale at the beginning of the trial and at the end of the trial. The model describes how this marker evolves over time in patients receiving standard-of-care and how covariates influence this. In this example, the relevant covariates are Age and Height (as described in the above cited paper by Wu et al.). It is to be noted that a person skilled in the art may also use other/further covariates when monitoring FEV1 progression. It is further to be noted that a person skilled in the art can apply the approach disclosed herein also to other diseases using other appropriate disease progression models. With regard to the FEV1 example, "Equation 3" of the Wu paper describes that the best prediction by the disease progression model for FEV1 was achieved by the following exponential function of age and height:

$$FEV_1 = \exp(theta_1 \times age + theta_2 \times height - theta_3) + theta_{drugeffect}$$

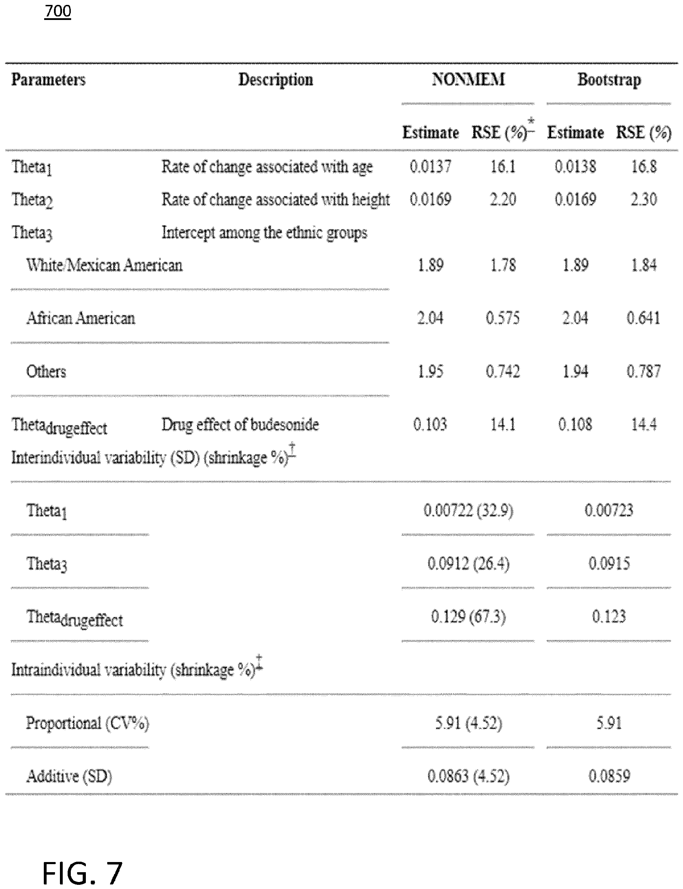

with $theta_1$ and $theta_2$ being the rate of change in FEV1 associated with age and height, respectively. The $theta_3$ refers to a baseline level for FEV1 (i.e., the FEV1 level at birth assuming the model is applicable to that age range). FIG. 7 reproduces table 2 of the Wu paper as table 700 showing the estimates of the parameters from the final model reflected by "Equation 3".

With this model at hand, the system 100 can predict how patients with a particular set of covariates (age and height) are expected to progress in terms of FEV1 over time, considering the variability between patients and the FEV1 measurement noise. It is to be noted that, in the herein described example, $theta_3$ is not considered as a relevant covariate of the disease progression model. This has the effect that, when using the disease progression model for simulating artificial patient data, the simulation relates to reference patients (not associated with a particular ethnic group). The variability between patients (interindividual variability) is described by equation 1 in Wu et al:

"Interindividual variability on $FEV_1$ was evaluated using an additive error model:

$$P_{ij} = P_{TVj} + \eta_{ij} \qquad (1)$$

where $P_{ij}$ is the true value of the $j$th parameter for the ith subject. $P_{TVj}$ is the population typical value (TV) for the $j$th parameter, and $\eta_{ij}$ is an interindividual random effect, which quantifies the deviation of $P_{ij}$ from $P_{TVj}$ and is assumed to follow a normal distribution with mean of 0 and variance of $\omega_{2j}$."

The FEV1 measurement noise (intraindividual variability or residual variability) is described by equation 2 in Wu et al:

"Intraindividual variability was evaluated using a combined proportional and additive error model as follows:

$$FEV_{1obs} = FEV_{1pred} \cdot (1 + \varepsilon_1) + \varepsilon_2 \qquad (2)$$

where $FEV_{1obs}$ are the observations and $FEV_{1pred}$ is the corresponding model prediction. $\varepsilon_1$ and $\varepsilon_2$ are independent and

normally distributed random variables with zero mean and variance of $\sigma 21$ and $\sigma 22$, respectively, that account for the residual unexplained variability. The final model was evaluated by a nonparametric bootstrap and the visual predictive check to assess the predictive performance and robustness."

The system 100 further receives 1200 a trial dataset 310 with trial covariates tc and clinical outcome data tco obtained from a plurality of human patients 300 participating in a target trial. The target trial comprises at least one treatment arm 301 with human patients receiving experimental treatment 11t (indicated by black heads in FIG. 1), and a control arm 302 with patients not receiving experimental treatment lint (indicated white heads in FIG. 1). The patients in the control arm 302 receive a placebo 10p instead of the experimental drug 10d. The considerations about the target trial and the clinical outcome regarding this example include an observation period of one year with the patients enrolled being randomly assigned to treatment or control arm, and then receiving the corresponding treatment (experimental drug or standard of care 10p) for a year. Enrollment of 300 patients per arm was intended. The patients were screened before randomization and their baseline FEV1 was measured. After the 1-year follow-up period, their FEV1 was measured again. The change in FEV1 during this period (FEV1 change from baseline) is the clinical outcome. As children grow, FEV1 is expected to increase. Therefore, change from baseline for patients not receiving the drug is expected to increase. Change from baseline for patients receiving the experimental drug is expected to be greater if the drug really works as it is intended to.

An artificial patient generator module 110 (APG) of the system 100 generates 1300 an artificial patient dataset 303 based on the data obtained from the target trial. In a first implementation, APG 110 uses distributions of the one or more corresponding covariates (e.g., age and height) and their correlations derived from the set of trial covariates tc to simulate artificial patient data based on variance-covariance matrices. This can be done by using the variance-covariance matrix of the covariates of interest obtained from the real patient's data of the target trial. In this example, the variance-covariance matrix for age and height had the form shown in the following Table 2:

Table 2 - variance-covariance matrix example

|  | Age | Height |
|---|---|---|
| **Age** | 18.69458 | 111.0345 |
| **Height** | 111.03450 | 719.4754 |

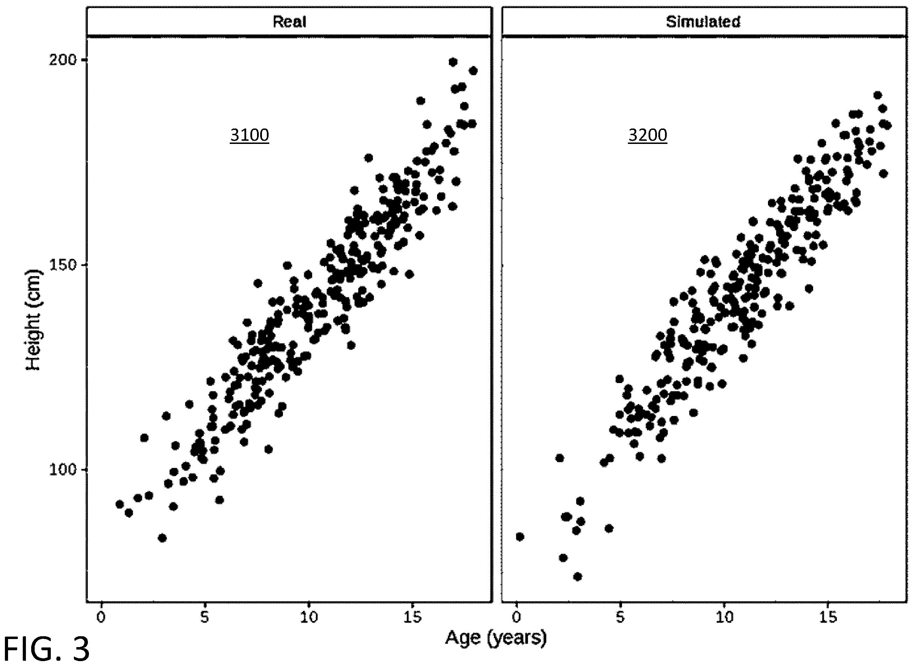

This variance-covariance matrix is then used to simulate the new cohort of the artificial patient dataset 303. In this implementation, the APG 110 has a filtering module APF 120 to filter the artificial patient dataset 303 in accordance with inclusion-exclusion criteria of the target trial so that data records associated with artificial patients which do not meet the inclusion criteria or which meet the exclusion criteria of the target trial are removed from the artificial patient dataset 303. Inclusion criteria are characteristics that the target trial participants must have if they are to be included in the clinical trial. Exclusion criteria are those characteristics that disqualify prospective participants from inclusion in the clinical trial. In the target trial, an exclusion criterion was that no patients above the age of 18 participated in the target trial. Therefore, artificial patients 303-1 and 303-2 which fall under such exclusion criterion (i.e., above 18 years-old) are filtered out and removed from the artificial patient dataset 303. FIG. 3 illustrates correlations of the covariates "Age" and "Height" (considered as relevant for the disease progression model) for the observed data 3100 which is used to build the variance covariance matrix, and for the simulated dataset 3200 which is created by using the variance-covariance matrix. The output of APG 110 is a dataset with simulated covariates sc' for the artificial patients of the artificial patient dataset 303.

In an alternative implementation, bootstrapping is used, where patients are randomly sampled from the target trial dataset, obtaining their paired values of age and height, to generate the simulated covariates sc' for the artificial patients. A large dataset can be generated (e.g., 10000 patients). In order to obtain such a big dataset out of a smaller set of real patients in the target trial sampling with replacement may be performed (meaning patients can be sampled more than once). In other words, by random sampling the trial covariates tc APG 110 generates an artificial patient data record for each patient of the artificial patient dataset 303 and stores the simulated covariates sc' of the respective artificial patient with said data record. Because sampling patients from the target trial automatically results in an artificial patient dataset which complies with the exclusion/inclusion criteria of the target trial, no further filtering is needed in this implementation of the APG 110.

The generated artificial patient dataset 303 with the simulated covariates sc' is now provided as input to the disease progression model 200. With this input, the disease progression model 200 generates 1400 a simulated dataset 320 with simulated covariates sc and simulated clinical outcome data sco for artificial patients not receiving experimental treatment. That is, the disease progression model simulates covariates and clinical outcomes only for artificial patients that would receive the standard-of-care, and therefore would belong to the control arm of the target trial. It is clear that the simulated patient data cannot relate to the treatment arm of the trial because it would be impossible to simulate the treatment effect of the drug. It is exactly the goal of the target trial to measure such treatment effect. In the example, clinical outcomes for 10000 artificial control patients were simulated. That is, the simulated dataset 320 now includes information about how each of the 10000 artificial patients evolves over time in terms of FEV1 progression.

A trial analyzer module (TA) 130 of system 100 finally determines 1500 the treatment effect 10d-te of the drug 10d. In a classic treatment effect analysis, only the trial dataset 310 (tc/tco) would have been used for such analysis. However, the herein disclosed method analyzes the trial dataset 310 and the simulated dataset 320 together using a power prior module 131 to incorporate the simulated dataset 320 as prior information. Thereby, the simulated dataset 320 is given a weight in comparison to the control arm of the trial dataset 310. The weight for the simulated dataset allows to acknowledge that the information gain obtained from an artificial patient is less than the information obtained from a real patient of the trail control arm 302. In other words, the weight is to be interpreted as the number of real patients that would have to be added to the trial control arm to provide the same amount of information as the simulated patients can contribute. The weight is a constraint (for TA 130 and its power prior function 131) which is limited by a user 1 to a maximum weight value 230-1 through a corresponding input to TA 130. The user 1 is setting this maximum weight value to be used by the treatment effect analysis algorithm based on medical considerations taking into account respective statistics.

For example, the maximum weight value 230-1 may be the number of patients used to develop the disease progression model 200. This reflects that the reliability of the disease progression model predictions is limited by the number of input datasets (of real patients) which were available for the model development. It would make no sense to give the total outcome of simulated patients a higher weight in the analysis than justifiable by the model which was used to generate the simulated clinical outcomes 320. In that sense, the number of patients used to develop the disease progression model provides an upper limit for the maximum weight value input 230-1. If for example, the disease progression model 200 was developed using a dataset of 660 real patients, the received maximum weight value 230-1 in this implementation (corresponding to an objective meaningful upper limit for the weight) is 660. That is, the 10.000 simulated patients are only given a weight in the analysis which corresponds to 660 patients in the control arm.

In another implementation, the maximum weight value 230-1 may be based on the similarity of patients used to develop the disease progression model 200 and target trial patients 300, and assumptions made to extrapolate from one to another population. In the FEV1 example, the disease progression model 200 was developed using data from adult trial patients. A multidisciplinary team of FEV1 experts reached an agreement that 660 adult patients are not as valuable to inform a pediatric trial as pediatric patients would be. Therefore, a 0.5 correction factor may be applied to account for the assumption made that the relation between age, height and FEV1 holds in pediatric patients, thus setting the maximum number of patients to 50% of the patients used to develop the model. This results in a maximum weight value of 330 patients. A reduction of the maximum weight value always results in a treatment effect 10d-te estimate which gets closer to the treatment effect that would be measured based on the trial dataset 310 alone. That is, the risk of introducing errors from the simulation is reduced, but at the same time the reliability of the measured treatment effect is reduced which will be shown further below.

In a third implementation, the user 1 may use a sensitivity analysis (SA) tool 230 to determine a maximum weight value which reduces the probability of concluding that the drug 10d works when it does not. In other words, the worst-case scenario is considered as a too high "false positive results probability" (i.e., the probability of concluding that the drugs works when it does not). In the FEV1 example, the simulated dataset 320 shows a lower FEV1 change from baseline than the patients 300 recruited in the trial. When pooling together the simulated data 320 of the artificial patients 303 and the real data 310 of the real patients 300, the FEV1 change from baseline in control patients could be estimated to be lower than the true FEV1 change from baseline. The consequence, when comparing control and treatment arms, would be an increased probability of concluding the drug increases FEV1 change from baseline, when it actually does not.

Under this worst-case scenario, a range of weights is evaluated through clinical trial simulation and analyzed using the power prior. FIG. 4 shows an example with six weight values in the range between 0 and 150 (units equivalent to number of patients) which was used for the FEV1 example. In this example, the user has decided for a threshold of 10% for the probability of false positives. That is, the maximum weight value to be used as input for TA 130 should keep the probability of a false positive outcome (Type I Error Rate) below 10%. By interpolation using linear regression, a weight of 60 was identified as the weight value which corresponds to a 10% Type I Error rate (0.1). In this case, the maximum weight value was set to 60 patients. As a result, in this example, the artificial patients cannot have a weight higher than 60 real patients in the analysis.

Other factors which can influence the selection of an appropriate maximum weight value input are: the precision of the estimates of the disease progression model, the results of model validation, and the size of the target trial. The skilled person knows how to assess these factors before entering the maximum weight value as input to TA 130. In general, if there is good concordance between observed and simulated control patients, a higher weight is desired, and when discrepancies occur, a lower weight is desired to prevent a biased analysis.

If the received maximum weight value 230-1 is the only consideration for setting the weight in the treatment effect analysis, then it would automatically become the predefined weight to be used by TA 130 in the analysis of the treatment effect 10d-te for said drug. In one embodiment, an automatic quantitative algorithm is used together with this maximum weight value to select the weight used for the analysis. In this embodiment, the used weight is further based on the similarity between the clinical outcome data sco for artificial patients not receiving experimental treatment and the clinical outcome data tco obtained from the control arm of the target trial. The similarity is obtained by using a dynamic borrowing method with a higher similarity leading to a higher dynamic borrowing weight value. The finally used weight value is the lower of the received maximum weight value and the obtained dynamic borrowing weight value.

An example of an algorithm implementing such a dynamic borrowing method is the dynamic borrowing for power prior analysis described Ibrahim JG, Chen MH, Gwon Y, Chen F. The power prior: theory and applications. Stat Med. 2015 Dec 10; 34(28):3724-49. This algorithm implements a cost function considering the difference between the clinical outcomes observed for the patients enrolled in the trial and the clinical outcomes of the patients in an external dataset. In the herein described method, the simulated dataset is used instead of the external dataset. Further, the algorithm uses the amount of information borrowed from the simulated dataset. This allows to reach a balance between the risk of introducing bias into the treatment effect analysis and the improvement of the estimate precision. Following Ibrahim et al. 2015, section 2, the cost function takes the following form:

$$\alpha_{0,PLC}^{opt} = \arg\min G(\alpha)$$

Where:

$$G(\alpha) = -2log[m^*(\alpha)] + \frac{log(n_0)}{\alpha}$$

Where:

$$m^*(\alpha) = \int L(\beta|D)L(\beta|D_0)^\alpha \pi_0(\beta)\, d\beta$$

Where:

- $\alpha$ = power parameter
- $n_0$ = sample size of external (artificial) control arm
- $\beta$ = response variable (clinical outcome)
- $D_0$ = observed outcomes in internal control arm
- D = observed outcomes in external (artificial) control arm
- $\pi_0$ = prior distribution of external data

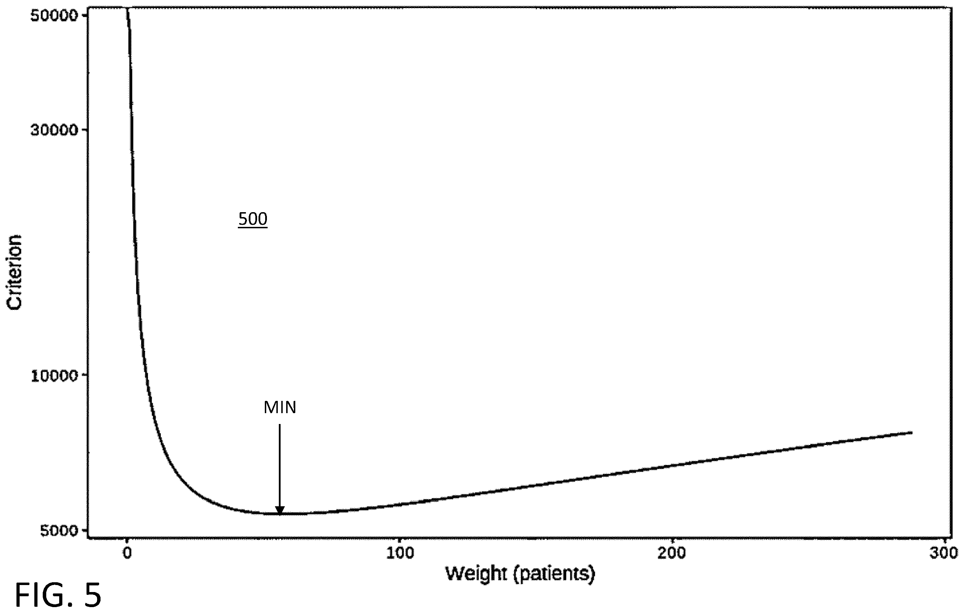

FIG. 5 illustrates the relationship of this cost-function 500 over a range of weights. For the FEV1 example, a minimum value MIN was found at the weight 58 (patients). As this weight was below the maximum weight value received from the user, the weight value determined by the dynamic borrowing is now used as the weight of simulated patients in the clinical trial analysis when using the power prior function PP 131 of TA 130. In case the dynamic borrowing had pointed to a weight above 60 patients, and the maximum weight value had been determined based on the third implementation as described in FIG. 4, the maximum weight value would have been used as the weight value given to the simulated patients for PP 131 because TA 130 always uses the lower weight value in this embodiment.

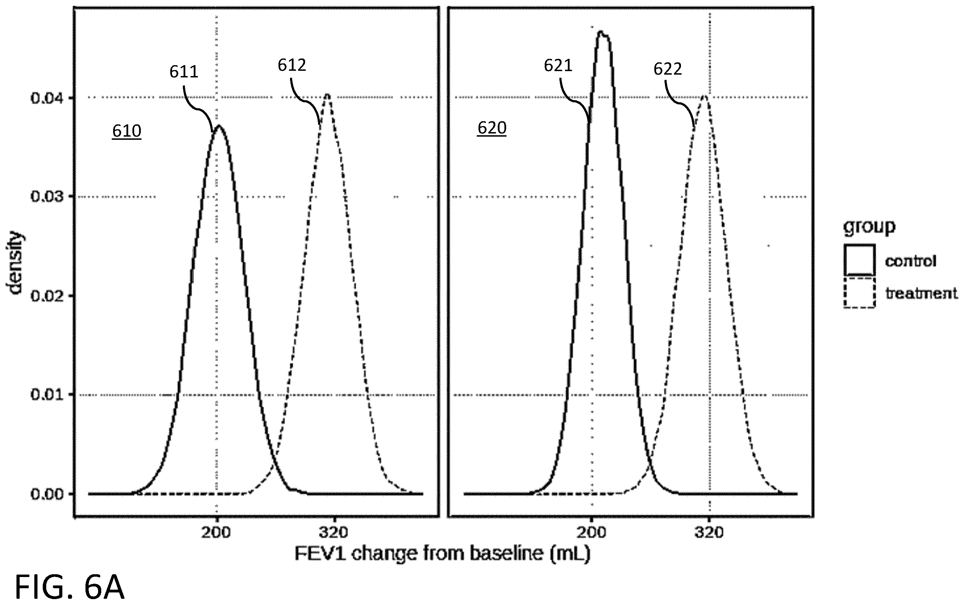

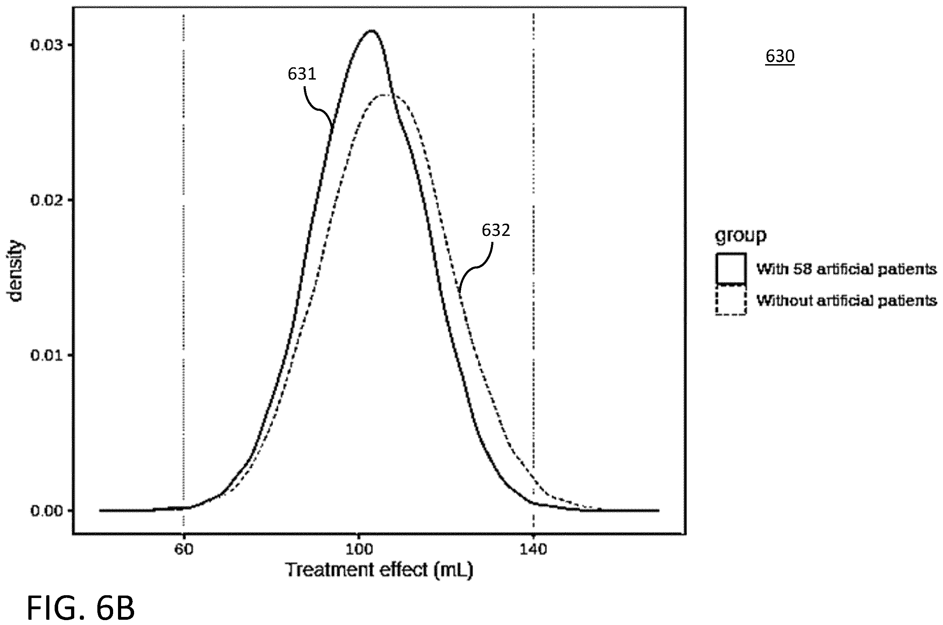

Turning back to FIG. 1, it is to be noted that the reliability of the determined treatment effect 10d-te is improved by using the PP 131 function of TA 130 to incorporate the clinical outcomes of the simulated patients with the appropriate weight in comparison to the classic clinical trial analysis which is only based on real patient data 310 from the clinical trial 300. This improvement effect is illustrated in FIGs. 6A, 6B by way of example for the FEV1 scenario.

Once the weight is given to the artificial patient data, the FEV1 change from baseline for the control arm in the trial is updated using the power prior 131 and the simulated FEV1 change from baselines. FIG. 6A shows the posterior distributions of FEV1 change from baseline estimate for the control arm (solid line) and the treatment arm (dashed line). The fact that the distribution of the treatment arm differs from the distribution of the control arm already indicates that there is a treatment effect associated with the medical drug. In the left chart 610, the distributions obtained with the classical treatment effect analysis without using the artificial patients is shown. The distribution 611 of the control arm is slightly lower and broader than the distribution 612 of the treatment arm. In the right chart 620, the distributions 621, 622 are obtained with the simulated patient data using PP 131 with the given weight value 58. That is, the control arm is enhanced by 58 simulated patients which results in more patients in the control arm than in the treatment arm. The distribution 621 of the control arm is now higher and narrower than the distribution 622 of the treatment arm (which remains of course unchanged vs. distribution 612). That is, the distribution 621 of the control arm with using the artificial patients with a weight of 58 leads to a better precision of the FEV1 change estimate.

FIG. 6B illustrates a chart 630 with the distributions 631, 632 of the treatment effects derived from the charts 610 and 620 of FIG. 6A. The distribution of the treatment effect is computed as the difference in FEV1 change from baseline between the respective treatment arm and control arm. The distribution 631 is derived from chart 620 (including 58 artificial patients). The distribution 631 is derived from chart 610 without artificial patients. FIG. 6B clearly demonstrates how the estimate of the treatment effect is improved by the herein disclosed approach using artificial patients in the control arm. The distribution 631 of the treatment effect measured by including artificial patients is narrower and has a higher peak - in short provides a more precise estimate of the treatment effect - than distribution 632 showing the distribution of the treatment effect derived from real patient data of the clinical trial alone. Hence, the proposed method not only improves the measurement of the treatment effect of a drug based on a clinical trial but further allows to achieve this improved result without adding additional patients to the control arm. This avoids unnecessary burden for such patients who would only receive a placebo and could not even profit from the treatment effect of the drug.

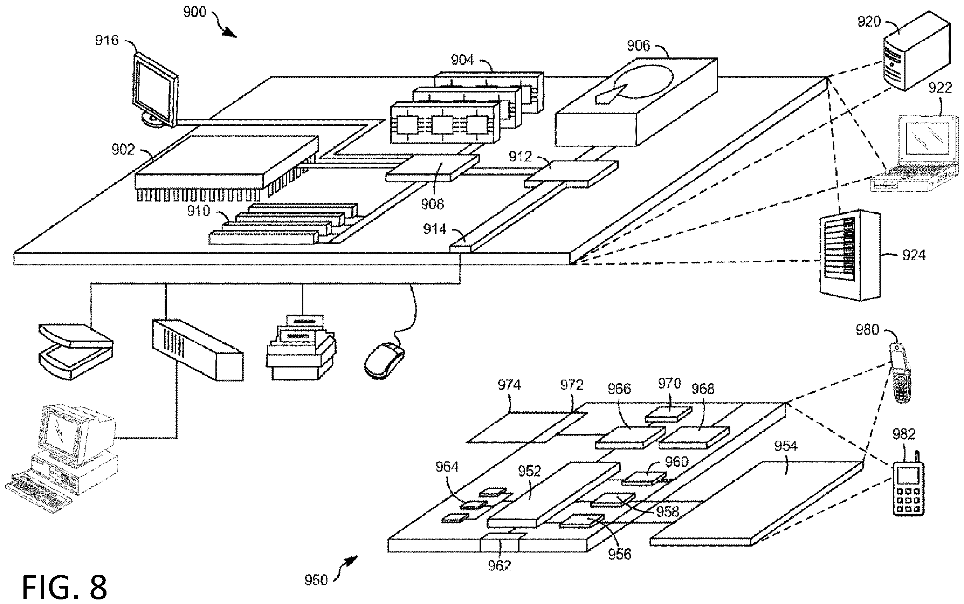

FIG. 8 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, servers, blade servers, mainframes, and other appropriate computers. Generic computer device 900 may correspond to the computer system 100 of FIG. 1. Computing device 950 is intended to represent various forms of mobile devices, such as personal digital assistants, cellular telephones, smart phones, and other similar computing devices. For example, computing device 950 may be used as a GUI frontend for a user to input the maximum weight value to the computer device 900, and in turn, receive from the computer device 9900, the predicted treatment effect of the drug. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low-speed interface 912 connecting to low-speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high-speed interface 908. In other implementations, multiple processing units and/or multiple buses may be used, as appropriate, along with multiple memories and types of memory. Also, multiple computing devices 900 may be connected, with each device providing portions of the necessary operations (e.g., as a server bank, a group of blade servers, or a processing device).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high-speed controller 908 manages bandwidth-intensive operations for the computing device 900, while the low-speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processing units. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the scope of the invention.

In addition, the logic flows depicted in the figures do not require the particular order shown, or sequential order, to achieve desirable results.

## Claims

1. A computer-implemented method (1000) for measuring the treatment effect of a medical drug (10d) for a disease, the method comprising:

   obtaining (1100) a disease progression model (200) of the disease, wherein the disease progression model (200) is based on historical covariates and clinical outcome data reflecting the progression of the disease for a plurality of patients affected by said disease, and wherein the disease progression model quantitively describes the time course (210) of disease progression by one or more corresponding covariates;

   receiving (1200) a target trial dataset (310) with trial covariates (tc) and clinical outcome data (tco) obtained from a plurality of human patients (300) participating in a target trial, wherein the target trial comprises at least one treatment arm (301) with human patients receiving experimental treatment (11t), and a control arm (302) with patients not receiving experimental treatment (lint);

   **characterized in that**
   generating (1300) an artificial patient dataset (303) by

   using distributions of one or more corresponding covariates and their correlations derived from the set of trial covariates (tc), and filtering the artificial patient dataset (303) in accordance with inclusion-exclusion criteria of the target trial so that data records associated with artificial patients (303-1, 303-2) which do not meet the inclusion criteria, or which meet the exclusion criteria of the target trial, are removed from the artificial patient dataset (303); or
   by random sampling the trial covariates (tc) with replacement, with each data record of the artificial patient dataset (303) storing covariates of a respective artificial patient;

   with the artificial patient dataset (303) as input for the disease progression model (200), generating (1400) a simulated dataset (320) with simulated covariates (sc) and simulated clinical outcome data (sco) for artificial patients not receiving experimental treatment;

   determining (1500) the treatment effect (10d-te) of the drug (10d) by analyzing the trial dataset (310) and the simulated dataset (320) together using a power prior approach (131) to incorporate the simulated dataset (320) as prior information, wherein the simulated dataset (320) is given a weight in comparison to the control arm of the trial dataset (310), with the weight based on at least a maximum weight value (230-1) received from a user (1).

2. The method of claim 1, wherein the given weight is further based on the similarity between the simulated clinical outcome data (sco) for artificial patients not receiving experimental treatment and the clinical outcome data (tco) obtained from the control arm of the target trial, the similarity being obtained by using a dynamic borrowing method with a higher similarity leading to a higher dynamic borrowing weight value, and wherein the given weight is the lower of the received maximum weight value and the obtained dynamic borrowing weight value.

3. The method of claim 1 or 2, wherein the maximum weight value is based on any of the following:

   the number of patients used to develop the disease progression model;
   the similarity of patients used to develop the disease progression model and target trial patients, and assumptions made to extrapolate from one to another population;
   a pre-determined risk of concluding that a drug works when it does not, obtained by using a sensitivity analysis performed for the disease progression model based on the historical data;
   the precision of the estimates of the disease progression model;
   the results of model validation; and
   the size of the target trial.

4. The method of any of the previous claims, wherein a clinical outcome is a measure referring to the occurrence of a disease, symptom, sign or laboratory abnormality, which constitutes the target outcome of clinical trials.

5. The method of claim 4, wherein the clinical outcome for a particular patient relates to one of the following: the time since the trial started until the disease progressed or the patient died; the loss of functional capacity of the lungs measured as the difference in volume that the patient can exhale at the beginning of the trial and at the end of the trial; Clinical Remission assessed by Mayo component sub-scores at week X, with a time frame of X weeks, with 12 weeks for induction and 52 weeks for remission; Clinical Remission at week X, with a time frame of X weeks, with 12 weeks for induction and 52 weeks for remission, or Enhanced Endoscopic Response at week X, with a time frame of X weeks with 12 weeks for induction and 52 weeks for remission; annual rate of decline in Forced Vital Capacity over X weeks in patients with SSc-ILD with a time frame of up to fifty-two weeks after the start of administration; improvement in fibrosis by at least one stage with no worsening of NASH, using NASH Clinical Research Network scoring system with a time frame of 18 months; the effect of a drug Y compared to placebo to achieve NASH resolution on liver histology in non-cirrhotic NASH patients, with a time frame: Measurements at Baseline and fifty-two weeks; change from Baseline in Best-Corrected Visual Acuity Score at the average of week 36 and week 40, as assessed using the Early Treatment Diabetic Retinopathy Study Visual Acuity Chart at a Starting Distance of 4 Meters, with time frame: Baseline to Week 40; and Survival or Progression-free survival with time frame: Z years.

6. The method of any of the previous claims, wherein one or more corresponding covariates comprise one or more of the following:

   one or more trends of the development of disease symptoms over time,
   the variability in disease progression between patients, and
   a quantitative description of the relationship between patient characteristics, disease characteristics, treatment characteristics and the disease progression dynamics.

7. The method of any of the previous claims, wherein a distribution of the treatment effect (10d-te) is computed as the difference in change from baseline of measured clinical outcome between the respective treatment arm and control arm.

8. A computer program product for measuring the treatment effect of a medical drug (10d) for a disease, the computer program product, when loaded into a memory of a computing device and executed by at least one processor of the computing device, causing the at least one processor to execute the steps of the computer-implemented method according to any one of the previous claims.

9. A computer system (100) for measuring the treatment effect of a medical drug (10d) for a disease, comprising:

   one or more interfaces (140) configured to obtain a disease progression model (200) of the disease, wherein the disease progression model (200) is based on historical covariates and clinical outcome data reflecting the progression of the disease for a plurality of patients affected by said disease, and wherein the disease progression model quantitively describes the time course (210) of disease progression by one or more corresponding covariates, and further configured to receive a target trial dataset (310) with trial covariates (tc) and clinical outcome data (tco) obtained from a plurality of human patients (300) participating in a target trial, wherein the target trial comprises at least one treatment arm (301) with human patients receiving experimental treatment (11t), and a control arm (302) with patients not receiving experimental treatment (lint);

**characterized in that**

an artificial patient generator module (110) configured to create an artificial patient dataset (303) by:

using distributions of the one or more corresponding covariates and their correlations derived from the set of trial covariates (tc), and filtering the artificial patient dataset (303) in accordance with inclusion-exclusion criteria of the target trial so that data records associated with artificial patients (303-1, 303-2) which do not meet the inclusion criteria, or which meet the exclusion criteria of the target trial, are removed from the artificial patient dataset (303); or

by random sampling the trial covariates (tc) with replacement, with each data record of the artificial patient dataset (303) storing covariates of a respective artificial patient;

the disease progression model (200) configured to generate a simulated dataset (320) with simulated covariates (sc) and simulated clinical outcome data (sco) for artificial patients not receiving experimental treatment, with the artificial patient dataset (303) as input;

a trial analyzer module (130) configured to determine the treatment effect (10d-te) of the drug (10d) by analyzing the trial dataset (310) and the simulated dataset (320) together using a power prior module (131) to incorporate the simulated dataset (320) as prior information, wherein the simulated dataset (320) is given a weight in comparison to the control arm of the trial dataset (310), with the weight based on at least a maximum weight value (230-1) received from a user (1).

10. The system of claim 9, wherein the given weight is further based on the similarity between the simulated clinical outcome data (sco) for artificial patients not receiving experimental treatment and the clinical outcome data (tco) obtained from the control arm of the target trial, the similarity being obtained by using a dynamic borrowing method with a higher similarity leading to a higher dynamic borrowing weight value, and wherein the given weight is the lower of the received maximum weight value and the obtained dynamic borrowing weight value.

11. The system of claim 9 or 10, wherein the maximum weight value is based on any of the following:

the number of patients used to develop the disease progression model;

a pre-determined risk of concluding that a drug works when it does not, obtained by using a sensitivity analysis performed for the disease progression model based on the historical data;

the similarity of patients used to develop the disease progression model and target trial patients, and assumptions made to extrapolate from one to another population;

the precision of the estimates of the disease progression model;

the results of model validation; and

the size of target trial.

12. The system of any of the claims 9 to 11, wherein a clinical outcome is a measure referring to the occurrence of a disease, symptom, sign or laboratory abnormality, which constitutes the target outcome of clinical trials.

13. The system of claim 12, wherein the clinical outcome for a particular human patient is any one of the following: the time since the trial started until the disease progressed or the human patient died; the loss of functional capacity of the lungs measured as the difference in volume that the human patient can exhale at the beginning of the trial and at the end of the trial; Clinical Remission assessed by Mayo component sub-scores at week X, with a time frame of X weeks, with 12 weeks for induction and 52 weeks for remission; Clinical Remission at week X, with a time frame of X weeks, with 12 weeks for induction and 52 weeks for remission, or Enhanced Endoscopic Response at week X, with a time frame of X weeks with 12 weeks for induction and 52 weeks for remission; annual rate of decline in Forced Vital Capacity over X weeks in patients with SSc-ILD with a time frame of up to fifty-two weeks after the start of administration; improvement in fibrosis by at least one stage with no worsening of NASH, using NASH Clinical Research Network scoring system with a time frame of 18 months; the effect of a drug Y compared to placebo to achieve NASH resolution on liver histology in non-cirrhotic NASH patients, with a time frame: Measurements at Baseline and fifty-two weeks; change from Baseline in Best-Corrected Visual Acuity Score at the average of week 36 and week 40, as assessed using the Early Treatment Diabetic Retinopathy Study Visual Acuity Chart at a Starting Distance of 4 Meters, with time frame: Baseline to Week 40; and Survival or Progression-free survival with time frame: Z year.

14. The system of any the claims 9 to 13, wherein one or more corresponding covariates comprise one or more of the following:

> one or more trends of the development of disease symptoms over time,
> the variability in disease progression between patients, and
> a quantitative description of the relationship between patient characteristics, disease characteristics, treatment characteristics and the disease progression dynamics.

15. The system of any of the previous claims 9 to 14, wherein a distribution of the treatment effect (10d-te) is computed as the difference in change from baseline of measured clinical outcome between the respective treatment arm and control arm.

**Patentansprüche**

1. Ein computerimplementiertes Verfahren (1000) zum Messen der Behandlungswirkung eines Medikaments (10d) für eine Krankheit, das Verfahren umfassend:

> Erstellen (1100) eines Krankheitsverlaufsmodells (200) der Krankheit, wobei das Krankheitsverlaufsmodell (200) auf historischen Kovariaten und klinischen Ergebnisdaten beruht, die den Verlauf der Krankheit für eine Vielzahl von Patienten widerspiegeln, die an dieser Krankheit leiden, und wobei das Krankheitsverlaufsmodell quantitativ die Zeitreihe (210) des Krankheitsverlaufs durch eine oder mehrere entsprechende Kovariaten beschreibt;
> Empfangen (1200) eines Target Trial-Datensatzes (310) mit Trial-Kovariaten (tc) und klinischen Ergebnisdaten (tco) von einer Vielzahl von menschlichen Patienten (300), die an einem Target Trial teilgenommen haben, wobei der Target Trial mindestens einen Behandlungsarm (301) mit menschlichen Patienten umfasst, die eine experimentelle Behandlung (11t) erhalten, und einen Kontrollarm (302) mit Patienten, die keine experimentelle Behandlung (11 nt) erhalten;
> **dadurch gekennzeichnet, dass**
> das Erstellen (1300) eines künstlichen Patienten-Datensatzes (303) durch
> das Verwenden von Verteilungen einer oder mehrerer entsprechender Kovariaten und ihrer Korrelationen, die von dem Satz der Trial-Kovariaten (tc) abgeleitet sind, und Filtern des künstlichen Patienten-Datensatzes (303) gemäß Inklusions-Exklusionskriterien des Target Trials, so dass Datenaufzeichnungen, die mit künstlichen Patienten (303-1, 303-2) verbunden sind, die die Inklsuionskriterien nicht erfüllen oder die die Exklusionskriterien des Target Trials erfüllen, aus dem künstlichen Patienten-Datensatz (303) entfernt werden; oder
> durch Zufallsauswahl der Trial-Kovariaten (tc) mit Austausch, wobei jede Datenaufzeichnung des künstlichen Patienten-Datensatzes (303) Kovariaten eines entsprechenden künstlichen Patienten speichert;
> wobei der künstliche Patienten-Datensatz (303) als Eingabe für das Krankheitsverlaufsmodells (200) einen simulierten Datensatz (320) mit simulierten Kovariaten (sc) erstellt (1400) und simulierte klinische Ausgabedaten (sco) für künstliche Patienten, die keine experimentelle Behandlung erhalten;
> Bestimmen (1500) der Behandlungswirkung (10d-te) des Medikaments (10d) durch gemeinsames Analysieren des Trial-Datensatzes (310) und des simulierten Datensatzes (320) unter Verwendung eines Power Prior-Ansatzes (131), um den simulierten Datensatz (320) als Vorinformation aufzunehmen, wobei dem simulierten Datensatz (320) eine Gewichtung im Vergleich zu dem Kontrollarm des Trial-Datensatzes (310) gegeben wird und wobei die Gewichtung auf mindestens einem maximalen von einem Benutzer (1) empfangenen Gewichtungswert (230-1) basiert.

2. Verfahren nach Anspruch 1, wobei die gegebene Gewichtung ferner auf der Ähnlichkeit zwischen den simulierten klinischen Ergebnisdaten (sco) für künstliche Patienten, die keine experimentelle Behandlung erhalten, und den klinischen Ergebnisdaten (tco), die aus dem Kontrollarm der Zielstudie erhalten wurden, basiert, wobei die Ähnlichkeit durch Verwenden eines dynamischen Aufnahme-Verfahrens erhalten wird, wobei eine höhere Ähnlichkeit zu einem höheren dynamischen Aufnahme-Gewichtungswert führt, und wobei die gegebene Gewichtung der niedrigere des empfangenen maximalen Gewichtungswerts und des erhaltenen dynamischen Aufnahme-Gewichtungswerts ist.

3. Verfahren nach Anspruch 1 oder 2, wobei der maximale Gewichtungswert auf Folgendem basiert:

Anzahl der Patienten, die für die Entwicklung des Krankheitsverlaufsmodells eingesetzt wurden;
Ähnlichkeit der Patienten, die für die Entwicklung des Krankheitsverlaufsmodells und der Target Trial-Patienten eingesetzt wurden, und aufgestellte Hypothesen, um von einer zur anderen Population zu extrapolieren;
einem vorbestimmten Risiko einer Schlussfolgerung, dass ein Medikament wirkt, wenn es nicht wirkt, das durch Verwenden einer für das Krankheitsverlaufsmodell auf Grundlage der historischen Daten ausgeführten Sensitivitätsanalyse erlangt wurde;
der Präzision der Schätzungen des Krankheitsverlaufsmodells;
der Resultate der Modellvalidierung; und
der Größe des Target Trial.

4. Verfahren nach einem der vorstehenden Ansprüche, wobei ein klinisches Ergebnis ein Maß ist, das sich auf das Auftreten einer Krankheit, eines Symptoms, Zeichens oder einer Laboranomalie bezieht, die das Zielergebnis der klinischen Studie bildet.

5. Verfahren nach Anspruch 4, wobei sich das klinische Ergebnis für einen bestimmten Patienten auf eines aus Folgendem bezieht: Die Zeit seit Trial-Beginn bis zum Fortschreiten der Krankheit oder dem Tod des Patienten; der Verlust der Funktionsfähigkeit der Lunge, gemessen als die Differenz im Volumen, das der Patient zu Beginn des Trials einatmen kann und dem Ende des Trials; klinische Remission, bewertet anhand von Mayo Komponenten-Subscores in Woche X, mit einem Zeitrahmen von X Wochen, mit 12 Wochen für Induktion und 52 Wochen für die Remission; klinische Remission in Woche X, mit einem Zeitrahmen von X Wochen, mit 12 Wochen für Induktion und 52 Wochen für Remission oder Erweiterte Endoskopische Reaktion in Woche X, mit einem Zeitrahmen von X Wochen mit 12 Wochen für Induktion und 52 Wochen für Remission; jährliche Verschlechterungsrate der forcierten Vitalkapazität über X Wochen bei Patienten mit SSc-ILD mit einem Zeitrahmen von bis zu zweiundfünfzig Wochen nach Beginn der Verabreichung; Verbesserung der Fibrose um mindestens eine Stufe ohne Verschlechterung der NASH, unter Verwendung des NASH-Scoring-Systems des Clinical Research Network mit einem Zeitrahmen von 18 Monaten; die Wirkung eines Medikaments Y im Vergleich zu einem Placebo, um eine Auflösung der NASH auf die Leberhistologie bei nicht zirrhotischen NASH-Patienten zu erreichen, mit einem Zeitrahmen: Messungen an der Baseline und zweiundfünfzig Wochen; Änderung von der Baseline der Sehschärfe bei bestmöglicher Korrektur durch eine Sehhilfe im Durchschnitt in Woche 36 und Woche 40, wie unter Verwendung des Early Treatment Diabetic Retinopathy Study Visual Acuity Chart bei einem Startabstand von 4 Metern bewertet, mit Zeitrahmen: Baseline bis Woche 40; und Überleben oder progressionsfreies Überleben mit Zeitrahmen: Z Jahre.

6. Verfahren nach einem der vorstehenden Ansprüche, wobei eine oder mehrere Kovariaten eines oder mehrere von Folgendem umfassen:

Eine oder mehr Entwicklungstendenzen der Krankheitssymptome über die Zeit, die Variabilität im Krankheitsverlauf zwischen Patienten, und
eine quantitative Beschreibung der Beziehung zwischen Patienteneigenschaften, Krankheitseigenschaften, Behandlungseigenschaften und der Dynamik des Krankheitsverlaufs.

7. Verfahren nach einem der vorstehenden Ansprüche, wobei eine Verteilung der Behandlungswirkung (10d-te) als Differenz der Veränderung von der Baseline des gemessenen klinischen Ergebnisses zwischen dem entsprechenden Behandlungsarm und dem Kontrollarm berechnet wird.

8. Computerprogramm-Produkt zum Messen der Behandlungswirkung eines Medikaments (10d) für eine Krankheit, wobei das Computerprogramm-Produkt, wenn es in einen Speicher eines Computergeräts geladen und von mindestens einem Prozessor des Computergeräts ausgeführt wird, den mindestens einen Prozessor veranlasst, die Schritte des computerimplementierten Verfahrens nach einem der vorstehenden Ansprüche auszuführen.

9. Computersystem (100) zum Messen der Behandlungswirkung eines Medikaments (10d) für eine Krankheit, umfassend:

Eine oder mehrere Schnittstellen (140), die konfiguriert sind, ein Krankheitsverlaufsmodell (200) der Krankheit zu erlangen, wobei das Krankheitsverlaufsmodell (200) auf historischen Kovariaten und klinischen Ergebnisdaten basiert, die den Verlauf der Krankheit für eine Vielzahl von Patienten, die von dieser Krankheit betroffen sind, widerspiegeln, und wobei das Krankheitsverlaufsmodell quantitativ den Zeitverlauf (210) des Krankheitsverlaufs durch eine oder mehrere entsprechende Kovariaten beschreibt, und ferner konfiguriert sind, einen Target Trial-Datensatz (310) mit Trial-Kovariaten (tc) und klinischen Ergebnisdaten (tco) zu erhalten, die von einer Vielzahl von menschlichen Patienten (300), die an einem Target Trial teilgenommen haben, erlangt wurden, wobei der Target Trial mindestens einen Behandlungsarm (301) mit menschlichen Patienten umfasst, die eine experimentelle Behandlung (11t) erhalten, und einen Kontrollarm (302) mit Patienten, die keine experimentelle Behandlung (11 nt) erhalten;

**dadurch gekennzeichnet, dass**

ein Generatormodul eines künstlichen Patienten (110) konfiguriert ist, durch Folgendes einen künstlichen Patienten-Datensatz (303) zu erstellen:

Verwenden von Verteilungen einer oder mehrerer entsprechender Kovariaten und ihrer Korrelationen, die von dem Satz der Trial-Kovariaten (tc) abgeleitet sind, und Filtern des künstlichen Patienten-Datensatzes (303) gemäß Inklusions-Exklusionskriterien des Target Trials, so dass Datenaufzeichnungen, die mit künstlichen Patienten (303-1, 303-2) verbunden sind, die die Inklusionskriterien nicht erfüllen oder die die Exklusionskriterien des Target Trials erfüllen, aus dem künstlichen Patienten-Datensatz (303) entfernt werden; oder durch Zufallsauswahl der Trial-Kovariaten (tc) mit Austausch, wobei jede Datenaufzeichnung des künstlichen Patienten-Datensatzes (303) Kovariaten eines entsprechenden künstlichen Patienten speichert;

das Krankheitsverlaufsmodell (200) ist dabei konfiguriert, einen simulierten Datensatz (320) mit simulierten Kovariaten (sc) und simulierten Ergebnisdaten (sco) für künstliche Patienten zu erzeugen, die keine experimentelle Behandlung erhalten, mit dem künstlichen Patienten-Datensatz (303) als Eingabe;

ein Trial-Analysemodul (130), das konfiguriert ist, die Behandlungswirkung (10d-te) des Medikaments (10d) durch Analysieren des Trial-Datensatzes (310) und des simulierten Datensatzes (320) zusammen mit der Verwendung eines Power-Prior-Moduls (131) zu bestimmen, um den simulierten Datensatz (320) als Vorinformation einzubeziehen, wobei dem simulierten Datensatz (320) eine Gewichtung im Vergleich zum Kontrollarm des Trial-Datensatzes (310) gegeben wird, wobei die Gewichtung auf mindestens einem maximalen Gewichtungswert (230-1) basiert, der von einem Benutzer (1) empfangen wird.

10. System nach Anspruch 9, wobei die gegebene Gewichtung ferner auf der Ähnlichkeit zwischen den simulierten klinischen Ergebnisdaten (sco) für künstliche Patienten, die keine experimentelle Behandlung erhalten, und den klinischen Ergebnisdaten (tco), die aus dem Kontrollarm des Target Trial erhalten wurden, basiert, wobei die Ähnlichkeit durch Verwenden eines dynamischen Aufnahme-Verfahrens erhalten wird, wobei eine höhere Ähnlichkeit zu einem höheren dynamischen Aufnahme-Gewichtungswert führt, und wobei die gegebene Gewichtung der niedrigere des empfangenen maximalen Gewichtungswerts und des erhaltenen dynamischen Aufnahme-Gewichtungswerts ist.

11. System nach Anspruch 9 oder 10, wobei der maximale Gewichtungswert auf Folgendem basiert:

Anzahl der Patienten, die für die Entwicklung des Krankheitsverlaufsmodells eingesetzt wurden; einem vorbestimmten Risiko einer Schlussfolgerung, dass ein Medikament wirkt, wenn es nicht wirkt, das durch Verwenden einer für das Krankheitsverlaufsmodell auf Grundlage der historischen Daten ausgeführten Sensitivitätsanalyse erlangt wurde; Ähnlichkeit der Patienten, die für die Entwicklung des Krankheitsverlaufsmodells und der Target Trial-Patienten eingesetzt wurden, und aufgestellte Hypothesen, um von einer zur anderen Population zu extrapolieren; der Präzision der Schätzungen des Krankheitsverlaufsmodells; der Resultate der Modellvalidierung und der Größe des Target Trials.

12. System nach einem der Ansprüche 9 bis 11, wobei ein klinisches Ergebnis ein Maß ist, das sich auf das Auftreten einer Krankheit, eines Symptoms, Zeichens oder einer Laboranomalie bezieht, die das Zielergebnis der klinischen Studie bildet.

13. System nach Anspruch 12, wobei sich das klinische Ergebnis für einen bestimmten menschlichen Patienten auf eines aus Folgendem bezieht: Die Zeit seit Trial-Beginn bis zum Fortschreiten der Krankheit oder dem Tod des Patienten; der Verlust der Funktionsfähigkeit der Lunge, gemessen als die Differenz im Volumen, das der Patient zu Beginn des Trials einatmen kann und dem Ende des Trials; klinische Remission, bewertet anhand von Mayo Komponenten-Subscores in Woche X, mit einem Zeitrahmen von X Wochen, mit 12 Wochen für Induktion und 52 Wochen für die Remission; klinische Remission in Woche X, mit einem Zeitrahmen von X Wochen, mit 12 Wochen für Induktion und 52 Wochen für Remission oder Erweiterte Endoskopische Reaktion in Woche X, mit einem Zeitrahmen von X Wochen mit 12 Wochen für Induktion und 52 Wochen für Remission; jährliche Verschlechterungsrate der forcierten Vitalkapazität über X Wochen bei Patienten mit SSc-ILD mit einem Zeitrahmen von bis zu zweiundfünfzig Wochen nach Beginn der Verabreichung; Verbesserung der Fibrose um mindestens eine Stufe ohne Verschlechterung der NASH, unter Verwendung des NASH-Scoring-Systems des Clinical Research Network mit einem Zeitrahmen von 18 Monaten; die Wirkung eines Medikaments Y im Vergleich zu einem Placebo, um eine Auflösung der NASH auf die Leberhistologie bei nicht zirrhotischen NASH-Patienten zu erreichen, mit einem Zeitrahmen: Messungen an der Baseline und zweiundfünfzig Wochen; Änderung von der Baseline der Sehschärfe bei bestmöglicher Korrektur durch eine Sehhilfe im Durchschnitt in Woche 36 und Woche 40, wie unter Verwendung des Early Treatment Diabetic Retinopathy Study Visual Acuity Chart bei einem Startabstand von 4 Metern bewertet, mit Zeitrahmen: Baseline bis Woche 40; und Überleben oder progressionsfreies Überleben mit Zeitrahmen: Z Jahre.

14. System nach einem der Ansprüche 9 bis 13, wobei eine oder mehrere entsprechende Kovariaten eines oder mehrere von Folgendem umfassen:

Eine oder mehr Entwicklungstendenzen der Krankheitssymptome über die Zeit, die Variabilität im Krankheitsverlauf zwischen Patienten, und
eine quantitative Beschreibung der Beziehung zwischen Patienteneigenschaften, Krankheitseigenschaften, Behandlungseigenschaften und der Dynamik des Krankheitsverlaufs.

15. System nach einem der vorstehenden Ansprüche 9 bis 14, wobei eine Verteilung der Behandlungswirkung (10d-te) als Differenz der Veränderung von der Baseline des gemessenen klinischen Ergebnisses zwischen dem entsprechenden Behandlungsarm und dem Kontrollarm berechnet wird.

**Revendications**

1. Procédé (1000), implémenté sur ordinateur, de mesure de l'effet thérapeutique d'un médicament (10d) pour une maladie, le procédé comprenant :

l'obtention (1100) d'un modèle de progression de maladie (200) de la maladie, le modèle de progression de maladie (200) étant basé sur des covariables historiques et des données de résultats cliniques reflétant la progression de la maladie pour une pluralité de patients atteints de ladite maladie, et le modèle de progression de maladie décrivant quantitativement l'évolution dans le temps (210) de la progression de la maladie par une ou plusieurs covariables correspondantes ;
la réception (1200) d'un ensemble de données d'essai (310) cible comportant des covariables d'essai (tc) et des données de résultats cliniques (tco) obtenues auprès d'une pluralité de patients humains (300) participant à un essai cible, l'essai cible comprenant au moins un bras traitement (301) comportant des patients humains recevant un traitement expérimental (11t), et un bras témoin (302) comportant des patients ne recevant pas de traitement expérimental (11nt) ;
**caractérisé par**
la génération (1300) d'un ensemble de données (303) de patients artificiels par

utilisation de distributions d'une ou de plusieurs covariables correspondantes et de leurs corrélations dérivées de l'ensemble de covariables d'essai (tc), et filtrage de l'ensemble de données (303) de patients artificiels conformément à des critères d'inclusion-exclusion de l'essai cible, de sorte que les enregistrements de données associés aux patients artificiels (303-1, 303-2) qui ne répondent pas aux critères d'inclusion, ou qui répondent aux critères d'exclusion de l'essai cible, sont éliminés de l'ensemble de données (303) de patients artificiels ; ou
par échantillonnage aléatoire des covariables d'essai (tc) avec remplacement, chaque enregistrement de données de l'ensemble de données (303) de patients artificiels mémorisant des covariables d'un patient artificiel respectif.

avec l'ensemble de données (303) de patients artificiels en tant qu'entrée pour le modèle de progression de maladie (200), la génération (1400) d'un ensemble de données simulé (320) comportant des covariables simulées (sc) et des données de résultats cliniques simulés (sco) pour les patients artificiels ne recevant pas de traitement expérimental ;
la détermination (1500) conjointe de l'effet thérapeutique (10d-te) du médicament (10d) par analyse de l'ensemble de données d'essai (310) et de l'ensemble de données simulé (320), à l'aide d'une approche Power Prior (131) pour incorporer l'ensemble de données simulé (320) en tant qu'informations antérieures, un poids étant attribué à l'ensemble de données simulé (320) par rapport au bras témoin de l'ensemble de données d'essai (310), le poids étant basé sur au moins une valeur de poids maximale (230-1) reçue d'un utilisateur (1).

**2.** Procédé selon la revendication 1, dans lequel le poids attribué est en outre basé sur la similarité entre les données de résultats cliniques simulés (sco) pour des patients artificiels ne recevant pas de traitement expérimental et les données de résultats cliniques (tco) obtenues à partir du bras témoin de l'essai cible, la similarité étant obtenue en utilisant une méthode d'emprunt dynamique, une plus grande similarité conduisant à une valeur plus élevée du poids d'emprunt dynamique, et le poids attribué étant la plus basse parmi la valeur de poids maximale reçue et la valeur de poids d'emprunt dynamique obtenue.

**3.** Procédé selon la revendication 1 ou 2, dans lequel la valeur de poids maximale est basée sur l'un(e) quelconque des suivant(e)s :

le nombre de patients utilisés pour développer le modèle de progression de maladie ;
la similarité de patients utilisés pour développer le modèle de progression de maladie et de patients de l'essai cible, et les suppositions faites pour extrapoler d'une population à une autre ;
un risque préétabli de conclure qu'un médicament est actif alors qu'il ne l'est pas, obtenu par utilisation d'une analyse de sensibilité effectuée pour le modèle de progression de maladie basé sur les données historiques ;
la précision des estimations du modèle de progression de maladie ;
les résultats de validation du modèle ; et
la taille de l'essai cible.

**4.** Procédé selon l'une quelconque des revendications précédentes, dans lequel un résultat clinique est une mesure se rapportant à l'occurrence d'une maladie, d'un symptôme, d'un signe ou d'une anomalie dans les résultats de tests de laboratoire, qui constitue le résultat cible d'essais cliniques.

**5.** Procédé selon la revendication 4, dans lequel le résultat clinique pour un patient particulier est en relation avec l'un(e) des suivant(e)s : le temps écoulé depuis le début de l'essai jusqu'à la progression de la maladie ou le décès du patient ; la perte de capacité fonctionnelle des poumons, mesurée en tant que la différence entre le volume que le patient peut exhaler au début de l'essai et celui à la fin de l'essai ; rémission clinique évaluée par des sous-scores composants de Mayo à la semaine X, avec un cadre temporel de X semaines, avec 12 semaines pour l'induction et 52 semaines pour la rémission ; rémission clinique à la semaine X, avec un cadre temporel de X semaines, avec 12 semaines pour l'induction et 52 semaines pour la rémission, ou réponse endoscopique accentuée à la semaine X, avec un cadre temporel de X semaines, avec 12 semaines pour l'induction et 52 semaines pour la rémission ; taux annuel de déclin de la capacité vitale forcée sur X semaines chez des patients atteints de SSc-ILD avec un cadre temporel de jusqu'à cinquante-deux semaines après le début de l'administration ; atténuation de fibrose par au moins un stade sans aggravation de NASH, en utilisant le système de cotation du réseau de recherche clinique sur la NASH avec un cadre temporel de 18 mois ; l'effet d'un médicament Y comparé à placebo pour atteindre la résolution de la NASH sur l'histologie hépatique chez des patients non cirrhotiques atteints de NASH, avec un cadre temporel : mesures à l'inclusion et à cinquante-deux semaines ; changement à partir de l'inclusion du score de meilleure acuité visuelle corrigée à la moyenne de la semaine 36 et de la semaine 40, tel qu'évalué à l'aide du tableau d'acuité visuelle dans l'étude de la rétinopathie diabétique avec traitement précoce à une distance initiale de 4 mètres, avec cadre temporel : de l'inclusion à la semaine 40 ; et survie ou survie sans progression avec cadre temporel ; Z ans.

**6.** Procédé selon l'une quelconque des revendications précédentes, dans lequel une ou plusieurs covariables correspondantes comprennent une ou plusieurs des suivantes :

une ou plusieurs évolutions du développement de symptômes de maladie au cours du temps,
la variabilité de la progression de la maladie parmi les patients, et
une description quantitative de la relation entre les caractéristiques des patients, les caractéristiques de la maladie, les caractéristiques du traitement et la dynamique de la progression de la maladie.

**7.** Procédé selon l'une quelconque des revendications précédentes, dans lequel une distribution de l'effet thérapeutique (10d-te) est calculée comme la différence de changement du résultat clinique mesuré, depuis l'inclusion, entre le bras traitement respectif et le bras témoin.

**8.** Produit programme informatique destiné à mesurer l'effet thérapeutique d'un médicament (10d) pour une maladie, le produit programme informatique, lorsqu'il est chargé dans une mémoire d'un dispositif informatique et exécuté par au moins un processeur du dispositif informatique, amenant ledit au moins un processeur à exécuter les étapes du procédé selon l'une quelconque des revendications précédentes, implémenté sur ordinateur.

**9.** Système informatique (100) destiné à mesurer l'effet thérapeutique d'un médicament (10d) pour une maladie, comprenant :

une ou plusieurs interfaces (140) configurées pour obtenir un modèle de progression de maladie (200) de la maladie, le modèle de progression de maladie (200) étant basé sur des covariables historiques et des données de résultats cliniques reflétant la progression de la maladie pour une pluralité de patients atteints de ladite maladie, et le modèle de progression de maladie décrivant quantitativement l'évolution dans le temps (210) de la progression de la maladie par une ou plusieurs covariables correspondantes, et configurées en outre pour recevoir un ensemble de données d'essai (310) cible comportant des covariables d'essai (tc) et des données de résultats cliniques (tco) obtenues auprès d'une pluralité de patients humains (300) participant à un essai cible, l'essai cible comprenant au moins un bras traitement (301) comportant des patients humains recevant un traitement expérimental (11t), et un bras témoin (302) comportant des patients ne recevant pas de traitement expérimental (11nt) ;

**caractérisé en ce**

**qu'**un module (110) générateur de patients artificiels est configuré pour créer un ensemble de données (303) de patients artificiels par :

utilisation de distributions desdites une ou plusieurs covariables correspondantes et de leurs corrélations dérives de l'ensemble de covariables d'essai (tc), et filtrage de l'ensemble de données (303) de patient artificiels conformément à des critères d'inclusion-exclusion de l'essai cible, de sorte que les enregistrements de données associés aux patients artificiels (303-1, 303-2) qui ne répondent pas aux critères d'inclusion, ou qui répondent aux critères d'exclusion de l'essai cible, sont éliminés de l'ensemble de données (303) de patients artificiels ; ou

par échantillonnage aléatoire des covariables d'essai (tc) avec remplacement, chaque enregistrement de données de l'ensemble de données (303) de patients artificiels mémorisant des covariables d'un patient artificiel respectif.

le modèle de progression de maladie (200) étant configuré pour engendrer un ensemble de données simulé (320) comportant des covariables simulées (sc) et des données de résultats cliniques simulés (sco) pour les patients artificiels ne recevant pas de traitement expérimental, avec l'ensemble de données (303) de patients artificiels en tant qu'entrée ;

un module analyseur d'essai (130) configuré pour déterminer l'effet thérapeutique (10d-te) du médicament (10d) par analyse conjointe de l'ensemble de données d'essai (310) et de l'ensemble de données simulé (320) à l'aide d'un module Power Prior (131) pour incorporer l'ensemble de données simulé (320) en tant qu'informations antérieures, un poids étant attribué à l'ensemble de données simulé (320) par rapport au bras témoin de l'ensemble de données d'essai (310), le poids étant basé sur au moins une valeur de poids maximale (230-1) reçue d'un utilisateur (1).

**10.** Système selon la revendication 9, dans lequel le poids attribué est en outre basé sur la similarité entre les données de résultats cliniques simulés (sco) pour des patients artificiels ne recevant pas de traitement expérimental et les données de résultats cliniques (tco) obtenues à partir du bras témoin de l'essai cible, la similarité étant obtenue en utilisant une méthode d'emprunt dynamique, une plus grande similarité conduisant à une valeur plus élevée du poids d'emprunt dynamique, et le poids attribué étant la plus basse parmi la valeur de poids maximale reçue et la valeur de poids d'emprunt dynamique obtenue.

**11.** Système selon la revendication 9 ou 10, dans lequel la valeur de poids maximale est basée sur l'un(e) quelconque des suivant(e)s :

le nombre de patients utilisés pour développer le modèle de progression de maladie ;
un risque préétabli de conclure qu'un médicament est actif alors qu'il ne l'est pas, obtenu par utilisation d'une analyse de sensibilité effectuée pour le modèle de progression de maladie basé sur les données historiques ;
la similarité de patients utilisés pour développer le modèle de progression de maladie et de patients de l'essai cible, et les suppositions faites pour extrapoler d'une population à une autre ;
la précision des estimations du modèle de progression de maladie ;
les résultats de validation du modèle ; et
la taille de l'essai cible.

**12.** Système selon l'une quelconque des revendications 9 à 11, dans lequel un résultat clinique est une mesure se rapportant à l'occurrence d'une maladie, d'un symptôme, d'un signe ou d'une anomalie dans les résultats de tests de laboratoire, qui constitue le résultat cible d'essais cliniques.

**13.** Système selon la revendication 12, dans lequel le résultat clinique pour un patient humain particulier est l'un quelconque des suivants : le temps écoulé depuis le début de l'essai jusqu'à la progression de la maladie ou le décès du patient humain ; la perte de capacité fonctionnelle des poumons, mesurée en tant que la différence entre le volume que le patient humain peur exhaler au début de l'essai et celui à la fin de l'essai ; rémission clinique évaluée par des sous-scores composants de Mayo à la semaine X, avec un cadre temporel de X semaines, avec 12 semaines pour l'induction et 52 semaines pour la rémission ; rémission clinique à la semaine X, avec un cadre temporel de X semaines, avec 12 semaines pour l'induction et 52 semaines pour la rémission, ou réponse endoscopique accentuée à la semaine X, avec un cadre temporel de X semaines, avec 12 semaines pour l'induction et 52 semaines pour la rémission ; taux annuel de déclin de la capacité vitale forcée sur X semaines chez des patients atteints de SSc-ILD avec un cadre temporel de jusqu'à cinquante-deux semaines après le début de l'administration ; atténuation de fibrose par au moins un stade sans aggravation de NASH, en utilisant le système de cotation du réseau de recherche clinique sur la NASH avec un cadre temporel de 18 mois ; l'effet d'un médicament Y comparé à placebo pour atteindre la résolution de la NASH sur l'histologie hépatique chez des patients non cirrhotiques atteints de NASH, avec un cadre temporel : mesures à l'inclusion et à cinquante-deux semaines ; changement à partir de l'inclusion du score de meilleure acuité visuelle corrigée à la moyenne de la semaine 36 et de la semaine 40, tel qu'évalué à l'aide du tableau d'acuité visuelle dans l'étude de la rétinopathie diabétique avec traitement précoce à une distance initiale de 4 mètres, avec cadre temporel : de l'inclusion à la semaine 40 ; et survie ou survie sans progression avec cadre temporel ; Z ans.

**14.** Système selon l'une quelconque des revendications 9 à 13, dans lequel une ou plusieurs covariables correspondantes comprennent une ou plusieurs des suivantes :

une ou plusieurs évolutions du développement de symptômes de maladie au cours du temps,
la variabilité de la progression de la maladie parmi les patients, et
une description quantitative de la relation entre les caractéristiques des patients, les caractéristiques de la maladie, les caractéristiques du traitement et la dynamique de la progression de la maladie.

**15.** Système selon l'une quelconque des revendications 9 à 14 précédentes, dans lequel une distribution de l'effet thérapeutique (10d-te) est calculée comme la différence de changement du résultat clinique mesuré, depuis l'inclusion, entre le bras traitement respectif et le bras témoin.

FIG. 1

FIG. 2

FIG. 4

FIG. 3

EP 4 295 369 B1

FIG. 5

EP 4 295 369 B1

FIG. 6A

EP 4 295 369 B1

FIG. 6B

EP 4 295 369 B1

700

| Parameters | Description | NONMEM | | Bootstrap | |
|---|---|---|---|---|---|
| | | Estimate | RSE (%)[*] | Estimate | RSE (%) |
| $Theta_1$ | Rate of change associated with age | 0.0137 | 16.1 | 0.0138 | 16.8 |
| $Theta_2$ | Rate of change associated with height | 0.0169 | 2.20 | 0.0169 | 2.30 |
| $Theta_3$ | Intercept among the ethnic groups | | | | |
| White/Mexican American | | 1.89 | 1.78 | 1.89 | 1.84 |
| African American | | 2.04 | 0.575 | 2.04 | 0.641 |
| Others | | 1.95 | 0.742 | 1.94 | 0.787 |
| $Theta_{drugeffect}$ | Drug effect of budesonide | 0.103 | 14.1 | 0.108 | 14.4 |
| Interindividual variability (SD) (shrinkage %)[†] | | | | | |
| $Theta_1$ | | 0.00722 (32.9) | | 0.00723 | |
| $Theta_3$ | | 0.0912 (26.4) | | 0.0915 | |
| $Theta_{drugeffect}$ | | 0.129 (67.3) | | 0.123 | |
| Intraindividual variability (shrinkage %)[‡] | | | | | |
| Proportional (CV%) | | 5.91 (4.52) | | 5.91 | |
| Additive (SD) | | 0.0863 (4.52) | | 0.0859 | |

FIG. 7

FIG. 8

EP 4 295 369 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2019220733 A1
- WO 2020072548 A1
- US 2015315651 A1

### Non-patent literature cited in the description

- **ROSIE YU.** Prediction of clinical responses in a simulated phase III trial of Crohn's patients administered the antisense phosphorothioate oligonucleotide ISIS 2302: comparison of proposed dosing regimens. *Antisense Nucleic Acid Drug Dev,* February 2003, vol. 13 (1), 57-66
- **COOK, S.F ; BIES, R.R.** Disease Progression Modeling: Key Concepts and Recent Developments. *Curr Pharmacol Rep,* 2016, vol. 2, 221-230
- **WU K ; GAMAZON ER ; LM HK ; GEELEHER P ; WHITE SR ; SOLWAY J et al.** Genome-wide interrogation of longitudinal FEV1 in children with asthma. *Am J Respir Crit Care Med,* 2014, vol. 190 (6), 619-27
- **M S CHATTERJEE et al.** Population Pharmacokinetic/Pharmacodynamic Modeling of Tumor Size Dynamics in Pembrolizumab-Treated Advanced Melanoma. CPT Pharmacometrics Syst. *Pharmacol.,* 2017, vol. 6, 29-39
- **VENKATESH PILLA REDDY et al.** Pharmacokinetic-pharmacodynamic modeling of antipsychotic drugs in patients with schizophrenia Part I: The use of PANSS total score and clinical utility. *Schizophrenia Research,* 2013, vol. 146 (1-3), 144-152
- **MARIO NAGASE et al.** Modeling Tumor Growth and Treatment Resistance Dynamics Characterizes Different Response to Gefitinib or Chemotherapy in Non-Small Cell Lung Cancer. *CPT Pharmacometrics Syst. Pharmacol,* 2020, vol. 9, 143-152
- **LU et al.** Poster presented at the Population Approach Group in Europe (PAGE) 21st Annual Meeting. *Population Analysis of wet-AMD Disease Progression and The Therapeutic Effect of Ranibizumab,* 05 June 2012, https://www.page-meeting.org/pdf_assets/8526-2012%20PAGE%20poster version_FINAL.PDF
- **CLARET et al.** Model-based prediction of phase III overall survival in colorectal cancer on the basis of phase II tumor dynamics. *J Clin Oncol,* vol. 27 (25), 4103-4108
- **BASILE et al.** Integrating disease progression models, non-clinical pharmacokinetic data and treatment response endpoints to optimize intravitreal dosing regimens. *Int J Clin Pharmacol Ther,* July 2014, vol. 52 (7), 574-586
- *Stat Med,* 10 December 2015, vol. 34 (28), 3724-49
- **IBRAHIM JG ; CHEN MH ; GWON Y ; CHEN F.** The power prior: theory and applications. *Stat Med.,* 10 December 2015, vol. 34 (28), 3724-49
- **IBRAHIM JG ; CHEN MH ; GWON Y ; CHEN F.** The power prior: theory and applications. *Stat Med,* 10 December 2015, vol. 34 (28), 3724-49